(19) Europäisches Patentamt
European Patent Office
Office européen des brevets

(11) Publication number: 0 193 414
A2

(12) # EUROPEAN PATENT APPLICATION

(21) Application number: 86301495.7

(22) Date of filing: 03.03.86

(51) Int. Cl.⁴: A 61 K 9/50

(30) Priority: 01.03.85 ZW 3385

(43) Date of publication of application: 03.09.86 Bulletin 86/36

(84) Designated Contracting States: AT BE CH DE FR GB IT LI LU NL SE

(71) Applicant: Fluck, David John
6, The Fold
Northwood Harare(ZW)

(72) Inventor: Fluck, David John
6, The Fold
Northwood Harare(ZW)

(74) Representative: Bentham, Stephen et al,
J.A. Kemp & Co. 14 South Square Gray's Inn
London WC1R 5EU(GB)

(54) **Liposomes, process for their preparation and compositions containing them.**

(57) The invention relates to liposomes which comprise a phospholipid and a trypanocidally active phenanthridine or diamidine compound or quinapyramine, processes for their preparation and pharmaceutical compositions containing them.

EP 0 193 414 A2

Croydon Printing Company Ltd.

0193414

# LIPOSOMES, PROCESS FOR THEIR PREPARATION AND COMPOSITIONS CONTAINING THEM

This invention relates to liposomes, processes for their preparation and compositions containing them. The liposomes according to the invention comprise a trypanocidally active phenanthridine or diamidine compound or quinapyramine.

Drug delivery systems incorporating liposomes are known and have been previously studied mainly with a view to usage by intravenous administration for the targeting of pharmacologically active agents to specific cells and tissues. However, as intravenously administered liposomes primarily localise in cells of the mononuclear phagocytic system of the liver, spleen and lung and are not able to enter or leave the circulation by penetration of the vascular capillary endothelial lining, the usefulness of such liposomes is limited. Liposomes have been investigated as a means of extending the clearance times of drugs and other active molecules from the circulation but although formulations which are cleared slowly from the circulation are known the clearance times remain inadequate for long term prophylaxis.

Some studies have been made of liposome distribution after subcutaneous administration and it has been demonstrated that small liposomes migrate from a subcutaneous injection site and enter the lymphatic circulation whereas larger liposomes are unable to do so.

In contrast the intramuscular route of liposome administration has not been extensively studied although it is known that a slow release of water soluble marker substances from injected muscle can be achieved by liposomal encapsulation. Knowledge of the pharmacokinetics of the absorption of drugs and other biologically active molecules from intramuscular liposomal drug depots is limited.

Phenanthridine compounds include several with trypanocidal activity and a number of veterinarily active formulations have been developed for field usage against animal trypanosomiasis and in particular against tsetse-fly transmitted African animal trypanosomiasis. The widespread emergence of drug-resistant strains of trypanosomes has however reduced the effectiveness of several of these phenanthridine trypanocides such that only 2,7-diamino-9-phenyl-10-ethylphenanthridinium (known as homidium), which as the chloride salt is marketed as Novidium and as the bromide salt as Ethidium, and Samorin, of which the active principle is 8-[(m-amidinophenylazo)amino]-3-amino-5-ethyl-6-phenylphenanthridinium chloride hydrochloride, now remain usable for the treatment and prophylaxis of the disease.

Homidium chloride and homidium bromide, have both therapeutic and prophylactic action. However the latter rarely extends for longer than 60 days and is commonly less where high tsetse fly challenge conditions apply. Reports of resistance to homidium chloride and bromide have appeared from many African countries and usage of this trypanocide

has been discontinued in some countries in favour of the non-phenanthridine compound diminazene aceturate (Berenil) resistance to which is, so far, minimal. Berenil also has babesicidal activity.

Samorin has therapeutic trypanocidal action at dosages of 0.25mg/kg but this drug is in the main used for prophylaxis of trypanosomiasis at dosages of 0.5-1.0 mg/kg. The period of protection obtained is dependant on the dosage administered and the level of tsetse fly challenge but is normally within the range 60-120 days.

Side effects in the form of liver damage, photosensitisation and severe local reaction were a feature of the usage of several of the earlier trypanocides and extreme toxicity precluded the further development of many active phenanthridine compounds into veterinarily acceptable trypanocides. Later formulations, which include homidium and Samorin, show considerably reduced toxicity although the subcutaneous route remains contraindicated in favour of administration into the less reactive muscle tissue and the neck is recommended as the site of injection in order to restrict drug residues to one part of the carcass. At the dosages used, that is 1 mg/kg for homidium and 0.5-1.0 mg/kg for Samorin, side effects from homidium are infrequent and from Samorin are seen mainly as a reaction by the injected tissue to the drug. This reaction is normally in the form of a painful, persistent, fibrosed, necrotic, occasionally fluid-filled lesion at the injection site.

Drug deposited in or on the muscle surrounding the lesion or within cells which have invaded the lesion forms a drug depot from which slow leakage of drug is considered to provide the long-term maintenance of blood drug levels required for prophylactic action. Drug depots are not observed after Berenil administration and those from homidium are in the form of a diffuse staining of muscle tissue with little indication of localisation in the tissue of significant amounts of drug. It is noteworthy that Berenil provides prophylactic cover for a few days only and that, although a longer period can be obtained from homidium administration, it is considerably less than that from Samorin and is very susceptible to breakdown under increased challenge conditions.

It is however a frequent observation that the tissue reaction to Samorin is more marked than described above and in particular that the fibrosis is not only of greater intensity at the actual injection site depot, which may become fully encapsulated and calcified, but also extends into the surrounding muscle. During a long prophylactic regime, which may apply for up to seven years under village conditions in Africa, and which involves the administration of Samorin every two-four months, the normal reaction becomes cumulative such that much of the neck tissue is occupied by drug depots. Where over-reaction has occurred fibrous tissue invasion of the neck muscles is extensive, neck movement and blood flow in the neck are

restricted, calcification of heavily fibrosed encapsulated drug depots is frequent, and soft tissue for subsequent injections is unavailable. There is thus created a situation whereby involuntary underdosage can readily occur as the result of partial drug release from encapsulated depots and reduced drug absorption due to the lessened blood flow. Furthermore due to the extensive development of fibrous tissue subsequent injections are rarely intramuscular in practice, are difficult to administer, and the chances of leakage to the extremely reactive subcutaneous tissues are increased. In this latter event there is a possibility of the entire depot being lost by sloughing. Furthermore tissue reaction may be considered to be the cause of low blood drug levels due to the non-presentation of livestock at the regular intervals required for a particular prophylactic regime in that when draught cattle on Samorin prophylaxis are required to work immediately after treatment the tissue reaction in the neck muscles is intensified by pressure from the yoke. Under such circumstances it is not uncommon for a treatment to be intentionally missed and hence for a particular treatment interval to be doubled.

It is known that the chances of drug resistant strains of trypanosomes emerging are increased by exposure of the parasites to low levels of trypanocides and hence in order to reduce this possibility it is essential that under-dosing, whether intentional or not, does not occur and

that treatment intervals relevant to local tsetse fly challenge conditions and parasite virulence are adhered to. Although this premise applies to the usage of all antiparasitic drugs it is particularly pertinent to those used for the treatment of African animal trypanosomiasis in that the limited number of drugs available for this purpose is unlikely to be increased in the foreseeable future.

Little information is presently available on those injection conditions which lead to minimal tissue reaction to intramuscular phenanthridine depots rather than to the described over-reaction. The manufacturers of Novidium and Samorin recommend that these drugs are administered in aqueous solution by slow, deep, intramuscular injection using a wide bore needle but even under laboratory conditions this has been shown to produce variable results. Under field conditions in which livestock handling facilities are limited and where adverse environmental and climatic conditions apply injection quality tends to be reduced. Furthermore both physical and physiological stress can be expected to adversely influence the injected animal's reaction to trypanocide administration.

There is, therefore a need for a trypanocide formulation which will localise as a depot after intra-muscular administration but to which reaction from the surrounding muscle tissue will be small and in particular will not include encapsulation of the depot and extensive fibrous tissue formation throughout the injected tissue.

Additionally, as the number of trypanocidal drugs available for the control of trypanosomiasis in livestock is limited, there is a need to increase and extend the efficacy of those drugs which are available.

The liposomes of the present invention provide, after intramuscular injection into animals a surprisingly long-lived depot of trypanocidally active phenanthridine or diamidine compound or quinapyramine. The production of such a depot affords a means of securing long term prophylaxis against trypanosomiasis. Adverse tissue reaction at the injection site (for example when the phenanthridine compound is Samorin) is reduced, with a reduction in the concomitant adverse effect on absorption of the trypanocide. The association of, for example, phenanthridine compounds such as Samorin, with the phospholipid used to prepare the liposomes is surprisingly tenacious and the stability of Samorin is thereby considerably improved.

The liposomes of the invention also make it possible to produce by intramuscular injection a depot of phenanthridine or diamidine compounds or quinapyramine which have, heretofore, not been known to form such depots to any significant extent such as homidium and Berenil.

The invention relates to a means of reducing the known side-effects of phenanthridine trypanocides by exploiting the lipid binding properties of these compounds and administering the drugs as liposomal forms of a lipid-drug complex. The liposomes are retained within

injected muscle tissue as a depot from which a slow-release of drug occurs and to which there is less reaction from the injected tissue. Furthermore it is possible to form intramuscular depots of some of the phenanthridine, diamidine and quinapyramine trypanocides which do not localise to any significant extent as intramuscular depots after administration as the free drug in aqueous solution. The invention thus offers a means of achieving prophylaxis from trypanocides which have heretofore only been useful for their curative effect and of extending the period of prophylaxis of those which, from aqueous solution, form only temporary depots.

The invention also provides a means of circumventing the known rapid lability in aqueous solution of some drugs, for example isometamidium chloride, as demonstrated by the recovery of undegraded isometamidium from liposomal formulations of this trypanocide after storage in aqueous suspension at ambient temperature for several weeks.

The present invention accordingly provides liposomes which comprise a phospholipid and a trypanocidally active phenanthridine or diamidine compound or quinapyramine.

The phenanthridine or diamidine compound or quinapyramine may be in aqueous solution contained in the compartments of the liposomes but is preferably present in the lipid lamellae of the liposomes of the invention.

The octanol-water partition coefficient of phenanthridine and diamidine trypanocides is intermediate between those of strongly hydrophobic and strongly hydrophilic substances and hence these compounds are not obviously suitable for incorporation and retention within liposomal lipid lamellae. Furthermore the binding of phenanthridines and diamidines and quinapyramine to lipid bilayers when these compounds are encapsulated within the aqueous compartments of liposomes is minimal and such liposomes leak phenanthridine or diamidine trypanocides readily although the leakiness can be decreased somewhat by inclusion of a negative charge in the liposomal membrane.

In preferred liposomes of the invention phenanthridine, diamidine or quinapyramine trypanocides are preferably complexed or bound to and incorporated within the lipid lamellae of large liposomes such that a significant amount of the trypanocide is retained for a pharmacologically useful time within the injected tissue by virtue of the physical retention there of the liposomal carrier and are during this time separated from close contact with the potentially reactive injected tissue by incorporation within the liposomal lipid lamellae.

Alternatively depot formation can be obtained from liposomes in which phenanthridine, diamidine or quinapyramine trypanocides are in solution in the aqueous compartments separating the lipid lamellae of large multi-lamellar liposomes or in the compartment enclosed by

the unilamellar liposome membrane. Depots established by this alternative procedure, which optionally include some free trypanocide, are useful as partial or temporary depots.

The liposomes according to the invention are preferably large to maximise retention of injected liposomes and not so small as to allow substantial leakage away from the site of injection: sizes in the range from 100nm to 15 microns are preferred, although larger liposomes are also suitable.

Phenanthridine compounds which may be incorporated in the liposomes of the invention include those shown in the following table:

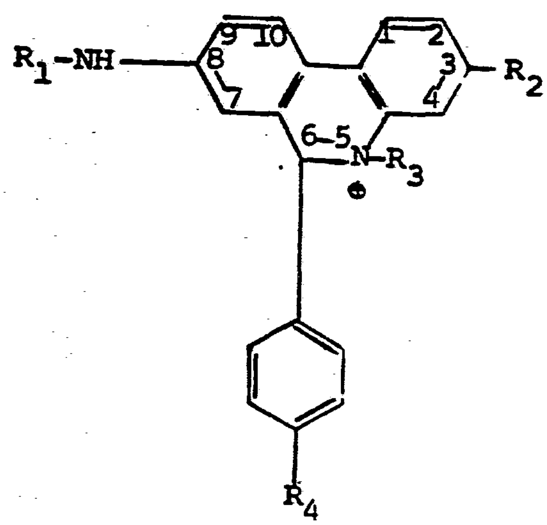

| | $R_1$ | $R_2$ | $R_3$ | $R_4$ |
|---|---|---|---|---|
| Phenidium | H | - | $CH_3$ | $NH_2$ |

| | | | | |
|---|---|---|---|---|
| Dimidium | H | $NH_2$ | $CH_3$ | - |
| Homidium | H | $NH_2$ | $C_2H_5$ | - |
| Pyrithidium (Prothidium) | 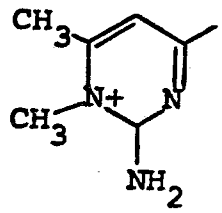 | $NH_2$ | $CH_3$ | - |
| Isometamidium | 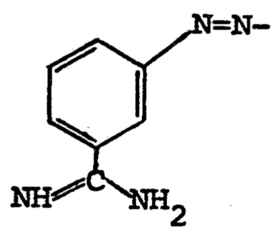 | $NH_2$ | $C_2H_5$ | - |

Preferred homidium salts are the chloride and bromide. Preferred isometamidium salts are the chloride and the chloride hydrochloride. Other suitable phenanthridine derivatives include those in which the groups $R_1$, $R_2$, $R_3$ and $R_4$ shown in the general formula depicted above are modified or replaced by other groups. 2,7-Di(m-amidinophenyldiazoamino)-10-ethyl-9-phenylphenanthridine salts (preferably the chloride dihydrochloride) are also suitable. This compound is described in Nature, 1961, pp 367, 368: the numbering system in the name of this phenanthridine compound does not correspond to that indicated in the formula above.

Suitable diamidine compounds include diminazine

salts, eg the aceturate (Berenil) of the formula:

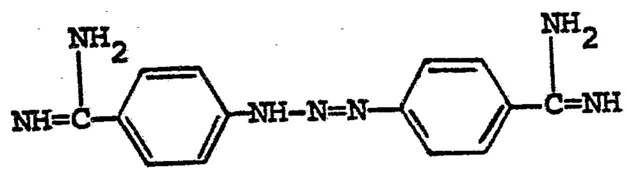

It will be seen that the chemical structure of Berenil is closely related to that of the phenanthridine derivative, isometamidium which contains a moiety of the Berenil molecule as a substituent represented by $R_1$.

The chemical structure of quinapyramine; ie 4-amino-6-[(2-amino-1,6-dimethyl-4-pyrimidinyl)amino]-1,2-quinoline, salts is also related to that of pyrithidium in that both contain the same substituted pyrimidinyl group.

The preferred phospholipid for use in the invention is lecithin (ie phosphatidyl choline). Other phosolipids which may be used include phosphatidyl ethanolamine and phosphatidyl serine.

The lipids used to prepare liposomes according to the invention may also comprise other materials. Such materials include:

a liposome membrane stabiliser, eg a sterol, preferably cholesterol; and

cationic or anionic adjuvants to produce a charge on the liposomal membrane, for example a phosphatidic acid which may be used to impart a negative charge to the liposomal wall, or stearylamine which may be used to impart

a positive charge thereto.

(A) According to a preferred feature of the invention liposomes are prepared by hydrating a composition comprising a phospholipid and a trypanocidally active phenanthridine or diamidine compound or quinapyramine, the composition being obtained by a process which comprises dissolving or dispersing a solid trypanocidally active phenanthridine or diamidine compound or quinapyramine in a liquid phospholipid.

In this preferred embodiment of the invention complexing of the trypanocide or granules thereof to the lipid is achieved by blending dry powdered trypanocide into a lipid in liquid form, for example soya bean lecithin, followed by hydration of the trypanocide-lipid complex by homogenisation in, for example, excess water, saline or an aqueous buffer solution. Hydration may also be effected using an aqueous solution of the same or a different trypanocide to yield liposomes in which trypanocide is present both in the lipid lamellae and in encapsulated aqueous solution.

In this embodiment it is further preferred that biologically acceptable emulsifiers be included in the composition to assist penetration of the aqueous swelling solution into the lipid-trypanocide complex. Such emulsifiers include Tween 60 and Span 20 which can be used separately, but which are preferably used together in a ratio of approximately 56 : 44 by weight and preferably

added, for example, to the liquid lipid in the proportion of approximately 185 parts by weight of lipid : 1 part by weight of emulsifier. Ratios of lipid to emulsifier from 75:1 to 200:1 by weight are particularly suitable. It is further preferred in this embodiment of the invention that the ratio by weight of the trypanocide, eg Samorin or Novidium, to the liquid lipid is approximately 1 : 10 by weight. The trypanocide commercially available as Berenil can be used as such in this method of the invention and, in that event, the ratio of the commercial product Berenil to liquid lipid is preferably approximately 1 : 5 by weight.

A composition comprising a phospholipid, a trypanocidally active phenanthridine or diamidine compound or quinapyramine and, preferably, an emulsifer, also constitutes a feature of the invention. Such a composition can be used to prepare lipsomes according to the invention. A composition comprising substantially solvent-free liquid lecithin, emulsifier and trypanocide, preferably in a ratio of approximately 10:0.054:1 by weight, is preferred.

In this preferred embodiment the blending of lipid, emulsifier and trypanocide can be achieved by mixing with a spatula or a paddle stirrer but can be undertaken by any process which allows the production of a homogeneous mixture from a viscous liquid and a dispersible powder.

Hydration and liposome formation from the trypanocide-lipid complex, which is facilitated by the presence of the emulsifier, is preferably carried out by

adding water, aqueous buffer or saline in the correct proportion for the final required concentration of liposomal trypanocide and homogenising the lipid and aqueous phases using, for example a Braun type 4-172 homogeniser at full speed for approximately 10 minutes. When the preferred features described above are adopted the liposome trypanocide preparation produced is generally seen by transmitted and fluorescent light microscopy primarily to comprise liposomes of approximately 1-10 m in diameter. Full incorporation of the trypanocide into the lipid is indicated after ultracentrifugation at 106,000 x g for 1 hr at 15°C in that less than 0.05% of the trypanocide is found in the aqueous supernatant. The leakage of phenanthridine or diamidine from liposomal trypanocides prepared by this preferred method of the invention and then stored in glass bottles at ambient temperature (ca 25°C) for 8 weeks is minimal for liposomal Samorin and acceptably low for liposomal-Novidium and liposomal-Berenil.

Liposomal-trypanocides prepared by this preferred method can be lyophilised by known methods and are found to be readily hydrated by replacement of the removed water. Such hydration is achieved by brief shaking by hand in the presence of a few 1mm diameter glass beads and does not require the use of a homogeniser or other source of mechanical agitation.

By the preferred method of this invention a tenacious complexing of phenanthridines or diamidines or

quinapyramine to lipids is achieved such that liposomes formed by hydration of the trypanocide-lipid complex retain trypanocide for an extended period and are ideal for the formation of tissue depots in which the trypanocide is dispersed, in liposomes, throughout the depot, is separated from potentially reactive tissue around the depot by the liposome lamellae and is amenable to release for subsequent absorption by dissolution of the liposomes or by migration or removal of the carrier liposomes from the depot.

The tissue depots formed after intra-muscular administration of liposomal-Samorin to goats and cattle are more discrete than those formed by administration of an aqueous solution of free Samorin and similar discrete depots have been obtained in goats from liposomal-Novidium and liposomal-Berenil. The latter trypanocide does not normally form a depot after intramuscular injection of free drug in aqueous solution.

(B) According to a further feature of the present invention liposomes are prepared by the removal of solvent from a solution comprising phospholipid and trypanocidally active phenanthridine or diamidine compound or quinapyramine to obtain a substantially solvent-free composition and hydration of the composition to produce liposomes.

In this embodiment of the invention binding of trypanocide to lipid is achieved by, for example, the mixing of a solution in a solvent, such as chloroform, of a phospholipid, which can be any phospholipid capable of

liposome formation below a temperature at which the trypanocide retains stability, with, for example a solution in a solvent such as methanol of the trypanocide, eg Samorin or homidium. The ratio of lipid to trypanocide is preferably approximately 10:1 by weight and, the ratio of chloroform and methanol when these solvents are used is approximately 7:1 by volume. Removal of the organic solvent by rotary evaporation of the solvent at a temperature below which the trypanocide loses stability and under vacuum results in a solvent-free lipid-trypanocide complex which can be subsequently hydrated to form liposomal-trypanocide for depot formation.

This embodiment is however not readily scaled-up to an industrial process and the solvent-free lipid-trypanocide complex is not as readily hydrated as that of the preferred method described above in (A). Liposomal-Samorin formed by the procedure (B) and including a proportion of radioactive Samorin as a tracer has been used to establish intramuscular depots of liposomal-Samorin in goats. The amount of radioactivity present in the blood plasma of these goats was compared with that in goats which had intramuscular depots formed from free Samorin and radioactive free-Samorin. It was observed that over a 100 day period the release of radioactivity from the liposomal Samorin depot was at a more constant rate and continued for longer than from the free Samorin depot. Furthermore reaction of the injected muscle as visualised by observation of the muscle and the lesion

under ultraviolet light and by assessment of the histopathology of the injection site, was found to be less to the liposomal-Samorin depot than to the free-Samorin depot.

The ratio of lipid to trypanocide in procedures (A) and (B) is preferably from 5:1 to 20:1 by weight, the ratio in the lamellae of the liposomes produced being substantially the same.

(C) According to a further feature of the invention liposomes are prepared by the hydration with an aqueous solution comprising a trypanocidally active phenanthridine or diamidine compound or quinapyramine of a phospholipid, preferably a liquid lecithin, or of a smectic mesophase deposited from solution. In the liposomes thus formed the aqueous solution of trypanocide is encapsulated and the trypanocide itself is not incorporated in the lipid to any substantial extent. The trypanocides leak readily from such liposomes and the proportion of the trypanocide in the hydration solution which is actually encapsulated is rarely higher than 20% by weight. Removal of the non-encapsulated trypanocide can be effected by known methods but is unlikely to be cost-effective for liposomal-trypanocides. However this embodiment, if the non-encapsulated drug is not removed, provides a means of achieving partial depot formation by the encapsulated solution and would allow, for example, both high blood levels of trypanocide for therapy, from the non-encapsulated trypanocide, and an element of

slow release for prophylaxis.

The 'liquid phospholipids' and liquid lecithin used in the procedures described in (A) and (B) should be capable of liquefaction at a temperature at which decomposition of the trypanocide is not substantial during preparation of the liposomes.

The expression phenanthridine or diamidine compound or quinapyramine as used in this specification and the accompanying claims is to be understood as embracing both the compounds themselves and salts thereof.

The invention is further described in the following Examples. In the Examples Novidium was used as the pure compound. References to Berenil and Samorin are to the commercially available products.

EXAMPLE 1

119.0 mg of Tween 60, 97.0 mg of Span 20 and 4.0g of powdered pure Novidium were in that order thoroughly mixed with 40.0 g of commercial, liquid, soya bean lecithin in a 1000 ml beaker using a spatula. 135 ml of distilled water were then added and the two phases were briefly mixed. It was noted that the water remained colourless indicating that all the Novidium was lipid-associated. The preparation was then homogenised using a Braun Type 4-172 domestic probe homogeniser operated at full speed for 10 minutes. On examination by fluorescent and transmitted light microscopy the presence of fluorescent liposomes in the size range of 1 - 10 microns was confirmed. Further water was then added to a final volume of 160 ml such that the concentration of Novidium was 2.5 per cent weight/volume. Ultra-centrifugation at 106,000 X g for 1 hour at 15°C produced a pellet of liposomal Novidium, a pale pink supernatant containing a minimal amount of free Novidium and some small liposomes, and a thin layer of oil and liposomes, to both of which Novidium was bound, on the surface.

A sample of the preparation stored in glass bottles at ambient temperature (28°C) for 15 days and then centrifuged at 106,000 X g for 1 hour at 15°C showed only a slight increase in colour in the supernatant indicating that leakage of Novidium was insignificant.

A goat culled 15 days after receiving an intramuscular injection of the preparation at a dose equivalent to 1 mg Novidium/kg live weight showed a discrete, intensely fluorescent depot of Novidium at the injection site and the presence of liposomal-Novidium within the depot.

EXAMPLE 2

The procedure of Example 1 was repeated except that 238 mg of Tween 60 and 194 mg of Span 20 were used.

The size range of the liposomes in this preparation was narrower than in that of Example 1 and in particular there were fewer liposomes larger than 10 microns in diameter.

EXAMPLE 3

The procedure of Example 2 was followed except that 4.0 g of Samorin was used and the amount of water used to hydrate and suspend the lipid was 160 ml. After homogenisation the volume was adjusted to 200 ml to give a 2% w/v concentration of Samorin in the liposomal form. Fluorescent and transmitted light microscopy showed a similar liposome preparation to that obtained from the preparation made in Example 2 except that the fluorescence colour was characteristic of Samorin.

A goat culled 15 days after the intramuscular administration of the preparation at an equivalent dose of

1 mg/kg live weight showed a discrete depot at the injection site and Samorin liposomes were found in the depot. An injection site depot from another goat to which free Samorin in aqueous solution was administered by the same route and at the same dosage as that from this Example was noted to be more diffuse.

Ultracentrifugation of the liposomal Samorin preparation produced in this Example was carried out on the freshly prepared material after 14 days storage at room temperature (28°C) and it was noted that the amount of Samorin in aqueous solution was less than 0.05% of the total added to the lipid; leakage of Samorin from the preparation was therefore minimal.

EXAMPLE 4

The procedure of Example 3 was followed using Berenil but in this case 468 mg of Tween 60, 382 mg of Span 20, 35.4 g of powdered Berenil granules and 78.75 g soya bean lecithin were used. After mixing as in the preceding Examples 140 ml of water were added and the preparation was homogenised as before. The volume was adjusted to 225 ml with water to produce a concentration of 7% active ingredient as used for free Berenil in aqueous solution. The liposomes formed in this Example were shown to be mainly up to 5 microns in diameter and ultracentrifugation at 106,000 X g for 60 minutes at 15°C which pelleted the

majority of the liposomal Berenil left a number in the supernatant indicating that some very small liposomes were formed from the lipid-Berenil complex by this procedure.

A goat injected as in the previous Examples but with liposomal Berenil at a dosage of 3.5 mg/kg live weight (calculated as active ingredient) showed on culling the presence of a discrete bright yellow depot of liposomal Berenil at one injection site and a less discrete, but entire, depot at a second injection site. Liposomal Berenil was recovered from each depot.

Examination of the injection tissue from another goat injected in the same way but with Berenil in solution at 3.5mg/kg failed to indicate the actual injection site and there was no indication of a depot.

EXAMPLE 5

4061 mg of Type X-E lecithin from the Sigma Chemical Company and 1005 mg cholesterol were dissolved in 200 ml of chloroform and 400 mg of Samorin in 29 ml of methanol to which was added 4 mg of tritiated Samorin (8.88 X $10^9$ dpm) was added to the lecithin solution. The solvents were removed by rotary evaporation under vacuum at 30°C and 100 ml of distilled water were added to the flask. The lipid was hydrated by hand shaking and vortex mixing which was facilitated by the addition of

approximately 50 1mm diameter glass beads and the partly hydrated preparation was left to swell at 4°C overnight prior to further hand shaking and vortex mixing the next day. The liposomal Samorin preparation produced by this procedure was examined by fluorescent and light microscopy and was found to contain liposomes up to 3 microns in diameter. The preparation was then centrifuged three times, with intermediate washing of the residue, at 48000 X g for 60 minutes at 15°C. The final supernatant was colourless. The first two supernatants were however the characteristic colour of an aqueous solution of Samorin and a considerable amount of Samorin remained unbound by the lipid in this procedure. Measurement of the amount of Samorin in the final residue showed that 22% of that added was retained in the liposome fraction.

Four goats received intramuscular injections of this preparation at a Samorin dosage of 0.5 mg/kg live weight and the amount of tritium in the plasma of these animals was measured in two for 15 days, one for 43 days and one to 100 days and was compared with that in the plasma of goats to which radioactive free Samorin in aqueous solution at the same dosage had been administered by the same route. It was found that the amount of radioactivity in the plasma of those goats which received the liposomal-Samorin formulation was at a more constant

level throughout the 100 day period than that from those which received the free Samorin.

Histopathology studies of the injection sites of liposomal Samorin and free Samorin-injected goats showed less reaction from the injected tissue to the liposomal Samorin than to the free Samorin.

The invention also provides pharmaceutical compositions which comprise liposomes according to the invention in association with a veterinarily acceptable carrier or diluent. Such compositions are preferably in a form which is suitable for intramuscular injection or which, after the addition of a suitable carrier or diluent, is suitable for intramuscular injection. The compositions may also contain trypanocidally active phenanthridine or diamidine compound in addition to that present in the liposomes.

The dosages of trypanocide in liposomal form are generally similar to these when the free trypanocide is administered.

CLAIMS

1. Liposomes which comprise a phospholipid and a trypanocidally active phenanthridine or diamidine compound or quinapyramine.

2. Liposomes according to claim 1 wherein the phospholipid is phosphatidyl choline.

3. Liposomes according to claim 1 or 2 which comprise, as trypanocidally active compound, 2,7-diamino-9-phenyl-10-ethylphenanthridinium bromide or chloride, 8-[(m-amidinophenylazo)amino]-3-amino-5-ethyl-6-phenylphenanthridinium chloride or the chloride hydrochloride, diminazine aceturate or quinapyramine.

4. Liposomes according to any one of the preceding claims in which the lipid lamellae of the liposomes comprise trypanocidally active compound.

5. Liposomes according to any one of the preceding claims wherein the phospholipid comprises a liposome membrane stabilizer.

6. Liposomes according to any one of the preceding claims which are from 100nm to 15 microns in diameter.

7. A process for the preparation of liposomes according to claim 1 which comprises:

(A) the hydration of a composition comprising a phospholipid and a trypanocidally active phenanthridine or diamidine compound or quinapyramine, the composition

being obtained by a process which comprises dissolving or dispersing a solid trypanocidally active phenanthridine or diamidine compound or quinapyramine in a liquid phospholipid;

(B) the removal of solvent from a solution comprising phospholipid and trypanocidally active phenanthridine or diamidine compound or quinapyramine to obtain a substantially solvent-free composition and hydration of the composition to produce liposomes; or

(C) the hydration with an aqueous solution comprising a trypanocidally active phenanthridine or diamidine compound or quinapyramine of a phospholipid or of a smectic mesophase deposited from solution.

8. A composition comprising a phospholipid and a trypanocidally active phenanthridine or diamidine compound or quinapyramine.

9. Pharmaceutical compositions which comprise liposomes according to any one of claims 1 to 6 in association with a veterinarily acceptable carrier or diluent.

10. Pharmaceutical compositions according to claim 9 which comprise trypanocidally active phenanthridine or diamidine compound or quinapyramine in addition to that present in the liposomes.